# EUROPEAN PATENT APPLICATION

(11) **EP 1 293 162 A2**
(43) Date of publication of application: **19.03.2003**
(21) Application number: 02019543.4
(22) Date of filing: 02.09.2002
(51) Int. Cl.: A61B 5/15

(54) **Needle for blood sampling with a safety shield**

(30) Priority: 17.09.2001 US 323186 P
(71) Applicant: Becton, Dickinson and Company, Franklin Lakes, New Jersey 07417-1880 (US)
(72) Inventor: Wilkinson, Bradley, North Haledon, New Jersey 07508 (US)
(74) Representative: von Kreisler, Alek, Dipl.-Chem.

(57) **Abstract**

The present invention is directed to a shieldable blood collection set. The shieldable blood collection set includes a fixture for connecting the blood collection set to a receptacle, an intravenous needle assembly including a needle cannula having a puncture tip, and a flexible tube extending between the fixture and the intravenous needle assembly. A shield assembly is in engagement with the needle assembly. The shield assembly is adapted for movement from a first position to a second position for protectively shielding the puncture tip of the needle cannula. The flexible tube includes a plurality of helical turns establishing a coiled structure. Such a coiled structure provides the blood collection set with an enlarged surface area for grasping by the palm of a user, thereby facilitating one-handed activation of the safety shield assembly.

## Description

### BACKGROUND OF THE INVENTION

### 1. Field of the Invention

The present invention relates to blood collection sets for safe and convenient handling of needles. More particularly, the present invention relates to a blood collection set including a needle assembly having a safety shield which is easily manipulated by a user.

### 2. Description of Related Art

Disposable medical devices having piercing elements are typically used for administering a medication or withdrawing a fluid, such as blood collecting needles, fluid handling needles and assemblies thereof. Current medical practice requires that the fluid containers and needle assemblies used in such systems be inexpensive and readily disposable. Consequently, existing blood collection systems, for example, typically employ some form of durable, reusable holder on which detachable and disposable needles and fluid collection tubes may be mounted. A blood collection system of this nature can be assembled prior to use and then disassembled after usage. Thus, these blood collection systems allow repeated use of the relatively expensive holder upon replacement of the relatively inexpensive needle and/or fluid collection tube. In addition to reducing the cost of collecting blood specimens, these blood collection systems also help minimize the production of hazardous medical waste.

A blood collection set or intravenous (IV) infusion set typically includes a needle cannula having a proximal end, a pointed distal end and a lumen extending therebetween. The proximal end of the needle cannula is securely mounted in a plastic hub with a central passage that communicates with the lumen through the needle cannula. A thin flexible thermoplastic tube is connected to the hub and communicates with the lumen of the needle cannula. The end of the plastic tube remote from the needle cannula may include a fixture for connecting the needle cannula to a blood collection tube or some other receptacle. The specific construction of the fixture will depend upon the characteristics of the receptacle to which the fixture will be connected.

In order to reduce the risk of incurring an accidental needle-stick wound, protection of used needle tips becomes important. With concern about infection and transmission of diseases, methods and devices to enclose the used disposable needle have become very important and in great demand. For example, needle assemblies commonly employ a safety shield that can be moved in a forward position into shielding engagement with a used needle cannula without risking an accidental needle stick.

Some needle shields are referred to as tip guards, and include a small rigid guard that can be forwardly telescoped along the length of a needle cannula and extended over the puncture tip of the needle for protection. Such conventional tip guard may include some form of tether for limiting the travel of the tip guard to the length of the needle cannula. Additionally, such conventional tip guard typically includes structure that lockingly engages over the tip of the used needle cannula to prevent a re-exposure of the needle. The structure for preventing re-exposure may include a metallic spring clip or a transverse wall integrally formed with one end of the tip guard.

Shielding of the needle in such assemblies typically involves grasping the needle hub with the flexible tubing in one hand and manipulating the needle shield into a forward blunting position with the other hand. Accordingly, activation of needle shields, and in particular forwardly blunting needle shields, typically requires two hands in order to properly shield the needle. Moreover, the small diameter of the flexible tubing attached to the needle assembly does not provide an adequate surface for handling.

Accordingly, a need exists for a needle assembly for use with a blood collection set which achieves secure and effective shielding of a used needle tip and which is easy to operate with a single hand.

### SUMMARY OF THE INVENTION

The present invention is directed to a blood collection set including a shieldable needle device. The shieldable blood collections set includes a fixture for connecting the blood collection set to a receptacle, an intravenous needle assembly including a needle cannula having a puncture tip, and a flexible tube extending between the fixture and the needle assembly. A shield assembly is in engagement with the needle assembly. The shield assembly is adapted for movement from a first position to a second position for protectively shielding the puncture tip of the needle cannula. The flexible tube includes a first end connected to the fixture and an opposed second end connected to the needle assembly, with a plurality of helical turns establishing a coiled structure between the fixture and the needle assembly. The coiled structure may include a circular cross-section, or may include an elliptical cross-section.

Desirably, the needle assembly includes a shield assembly adapted for movement from a first position to a second position for protectively shielding the puncture tip of the needle cannula. The shield assembly may include a housing extending about the needle cannula, and adapted for movement from the first position to the second position for protectively shielding the puncture tip of the needle cannula. The housing may include a lip portion adapted for engagement with a user's thumb for effecting movement from the first position to the second position.

Also, the needle assembly may include a hub, with the needle cannula extending from one end of the hub and the flexible tube attached to an opposing end of the hub. Such a hub desirably includes a pair of wings extending laterally from opposing sides thereof. The housing of the shield assembly may further include grooves extending laterally through the housing for accommodating the wings of the hub therethrough.

The coiled structure is desirably adjacent the shield assembly such that the shield assembly can be moved to the second position by a user's hand while maintaining the coiled structure in the palm of the user's hand. In this manner, one-handed activation of the safety shield assembly can be accomplished through the enlarged surface area established through the coiled structure for grasping by the palm of a user.

### DESCRIPTION OF THE DRAWINGS

FIG. 1 is a perspective view of a fully assembled blood collection set in accordance with the present invention with a packaging cover thereon;

FIG. 2 is a top plan view of the shieldable needle assembly of the blood collection set of FIG. 1 in a retracted position;

FIG. 3 is a cross-sectional view taken along line 3-3 of FIG. 2;

FIG. 4 is a cross-sectional view in an alternate embodiment;

FIG. 5 is a perspective view of the blood collection set shown in use; and

FIG. 6 is a perspective view of the shieldable needle assembly in an extended shielded position.

### DETAILED DESCRIPTION

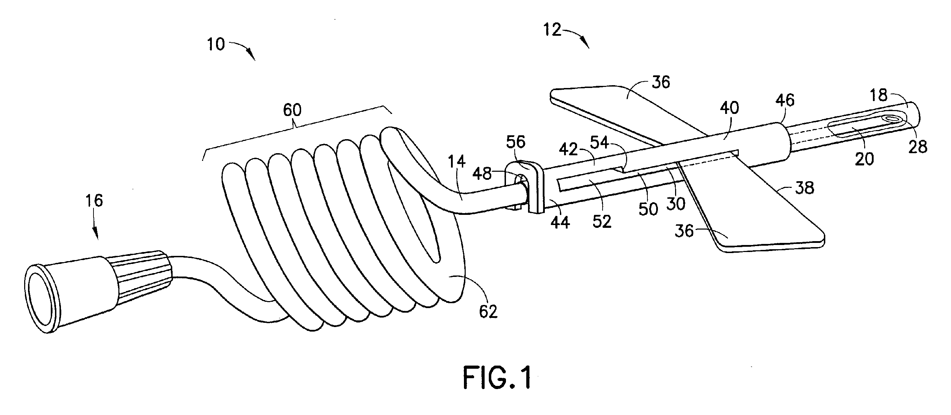

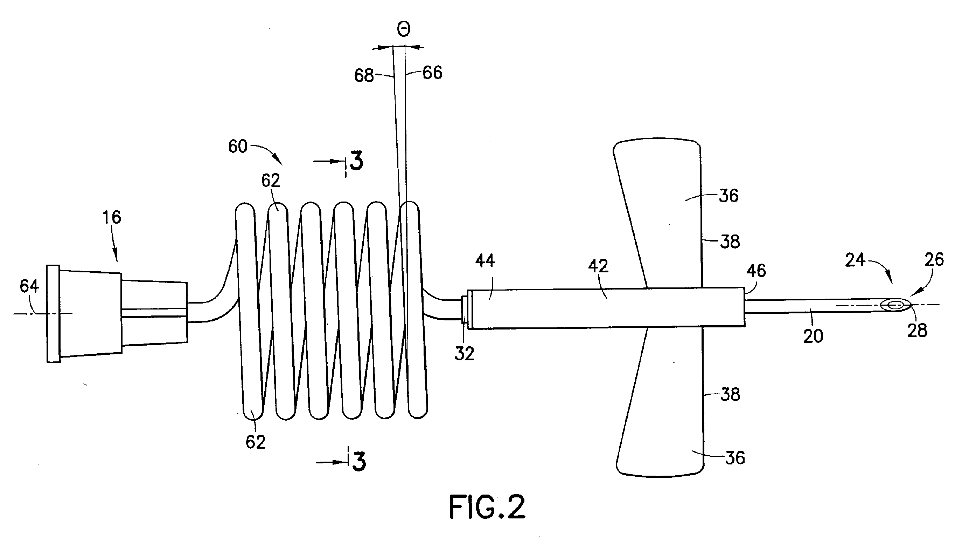

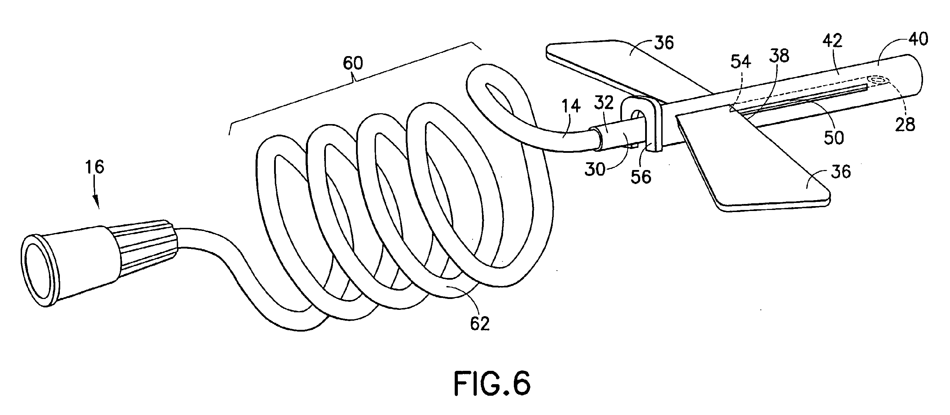

Referring to the drawings in which like reference characters refer to like parts throughout the several views thereof, FIGS. 1-6 illustrate a blood collection set in accordance with the present invention and the related features. As shown in FIGS. 1 and 2, blood collection set **10** includes a shieldable needle device **12,** a flexible tube **14** extending from needle device **12,** a fixture **16** mounted to tube **14** and a packaging cover **18** removably mounted to portions of needle device **12** opposite tube **14,** such as through a frictional engagement.

Shieldable needle device **12** may be any needle device including means for shielding an exposed needle tip, as is known in the art. Desirably, needle device **12** is a forward shielding needle device, in which a shielding member is moved from a first position in which the tip of the needle is exposed, to a second position encompassing the tip of the needle to shield the needle tip. For example, as shown in FIGS. 1 and 2, shieldable needle device **12** of blood collection set **10** includes a needle cannula **20,** a hub **30,** and a shield assembly **40.** While the present invention is described in terms of a blood collection set incorporating needle device **12,** it is understood that various other needle devices may be apparent to those skilled in the art for use in connection with the blood collection set of the present invention.

Needle cannula **20** includes a proximal end and an opposing distal end **24,** with lumen **26** extending through needle cannula **20.** Distal end **24** of needle cannula **20** is beveled to define a sharp puncture tip **28,** such as an intravenous puncture tip. Puncture tip **28** is provided for insertion into a patient's blood vessel, such as a vein, and is therefore designed to provide ease of insertion and minimal discomfort during venipuncture.

Needle assembly **12** further includes hub **30.** Hub **30** is a unitary structure, desirably molded from a thermoplastic material. Hub **30** is defined by a rigid tubular wall **32** extending from a proximal end to distal end thereof. Tubular wall **32** is characterized by an internal passage extending therethrough from the proximal end to the distal end of hub **30.**

Hub **30** further includes a pair of stabilizers in the form of wings **36** extending laterally from tubular wall **32** at opposing sides thereof. Wings **36** provide hub **30,** and needle assembly **12,** as a butterfly-type wing assembly, assisting in positioning and placement of needle assembly **12** and blood collection set **10** during a blood collection procedure.

Needle cannula **20** is positioned within the internal passage of hub **30,** and extends from the distal end of hub **30.** Desirably, needle cannula **20** and hub **30** are separate parts which are fixedly attached and secured through an appropriate medical grade adhesive or the like.

Needle assembly **12** further includes shield assembly **40.** Shield assembly **40** is slidably telescoped for axial movement along needle assembly **12** between a first proximal position, in which needle cannula **20** is at least partially exposed, and a second distal position adjacent puncture tip **28,** in which needle cannula **20** and puncture tip **28** are encompassed by shield assembly **40.**

Shield assembly **40** includes a housing **42.** Housing **42** is a unitary structure, desirably molded from a thermoplastic material, including a proximal end **44,** a distal end **46,** and an internal passage **48** extending between the ends. Shield assembly **40** further includes a pair of grooves **50** extending through housing **42** along opposing sides thereof. Grooves **50** are adapted to accommodate wings **36** of hub **30** extending through housing **42,** during movement of shield assembly **40** from the first position to the second position. Grooves **50** of shield assembly **40** further include an enlarged portion, such as slots **52,** adjacent proximal end **44** of housing **42** and extending through housing **42.** Shield assembly **40** further includes a lip **56** at a proximal end thereof. Lip **56** provides an extending surface for engagement with a user's thumb in order to activate shield assembly **40** for forward movement between the first position and the second position, as will be discussed.

It is noted that shield assembly **40** may be any type of shield assembly which is capable of forward movement to a shielding position to encompass needle cannula **20.** In particular, shield assembly **40** may include any blunting or shielding mechanism, which is adapted for movement along needle cannula **20** when shield assembly **40** is moved from the proximal position to the distal position, and which extends over puncture tip **28** of needle cannula **20** when shield assembly **20** is moved to the fully shielded distal position.

For example, housing **42** of shield assembly **40** may include an end cap having a blocking surface that is slidably telescoped on needle cannula **20** for axial movement from the proximal position to the distal position, where the end cap protectively surrounds distal end **24** of needle cannula **20.** Additionally, the end cap may be configured to prevent proximal movement after the blocking surface of the end cap has advanced sufficiently in a distal direction to protectively enclose puncture tip **28** of needle cannula **20.** The end cap may be in the form of a tip guard, which may include a tip guard housing formed from a plastic material, and a protective clip, such as a metallic spring clip mounted to the housing. In such an arrangement, the clip can be biased against the needle cannula when the tip guard is in the proximal position and can be resiliently moved over the distal end and distal tip of the needle cannula when the tip guard is in the distal position, thereby preventing piercing by the tip of the needle. Other shield assemblies will be apparent to those skilled in the art.

As noted, blood collection set **10** further includes flexible tube **14** extending between needle device **12** and fixture **16.** Flexible tube **14** includes a first end connected to needle device **12,** and a second end connected to fixture **16.** Fixture **16** is provided for connecting blood collection set **10** to a receptacle, for example a non-patient needle assembly and blood collection tube, as is known in the art.

Flexible tube **14** further includes a coiled structure in the form of coil **60.** In particular, flexible tube **14** includes a plurality of turns **62** extending helically from one end of flexible tube **14** to the other end. Such plurality of helical turns **62** provides flexible tube **14** with an enlarged surface area, thereby defining the shape of coil **60.** Such an enlarged surface area provides for better tactile engagement with the user's hand during activation of shield assembly **40,** as will be discussed in further detail herein.

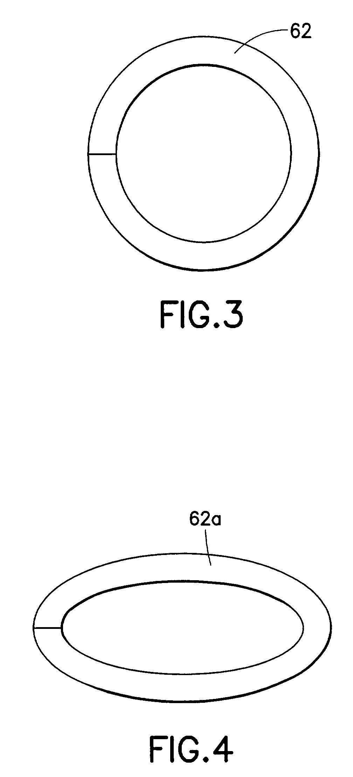

FIG. 3 depicts a cross-section of one turn **62** of coil **60.** As can be seen, coil **60** includes a substantially circular cross-section, providing coil **60** with an enlarged surface area as compared with the diameter of a linear portion of flexible tube **14** in an uncoiled state. Moreover, FIG. 2 demonstrates the helical nature of turns **62** of coil **60.** In particular, flexible tube **14** coils about axis **64** of blood collection set **10,** defining coil **60** extending generally axially along axis **64** of blood collection set **10.** Each of turns **62** extends circumferentially about axis **64,** in a generally helical direction in a series of planes which are substantially parallel to a cross-section of axis **64.** The helical nature of coil **60** is defined by angle θ, which defines the slope of the helical turns with respect to line **66,** which represents a perpendicular cross-section of axis **64.** When angle θ is a small angle approaching 0° and helical turns **62** are tightly wound, coil **60** includes a generally circular cross-section, as depicted in FIG. 3. Moreover, when angle θ is a larger angle increasing away from 0°, for example, toward 45°, a circular cross-sectional profile can be maintained, if helical turns **62** are spaced apart from each other and not maintained in a tightly wound manner in contact with each other. On the other hand, when angle θ is a larger angle increasing away from 0°, for example, toward 45°, and helical turns **62** remain tightly wound or in contact with each other, the slope of coil **60** increases, with coil **60** including a generally elliptical cross-section, as depicted in the alternate embodiment of FIG. 4. Such an elliptical cross-section provides coil **60** with a generally tubular elliptical profile for grasping by a user's hand. Such an elliptical profile can alternatively be provided by providing the coil in a generally oval helix, as opposed to a generally circular helix.

Activation of shield assembly **40** is accomplished by exerting a force on housing **42** in the direction of arrow **80** while maintaining hub **30** (and needle cannula **20** attached thereto) in place, thereby causing housing **42** to move along needle cannula **20** in the direction of arrow **80** toward distal end **24** and toward puncture tip **28.** Wings **36** of hub **30** extend through housing **42** of shield assembly **40.** During such movement of housing **42** along needle cannula **20,** wings **36** slide within grooves **50** of housing **42.** As such, housing **42** moves from the proximal position to the distal position. When in the distal position, wings **36** travel within grooves **50** to slots **52,** which represent an enlarged section of grooves **50.** Slots **52** include a shoulder **54** on a distal edge thereof. Once wings **36** are positioned within slots **52,** the forward edge **38** of wings **36** abuts shoulder **54** of slots **52** in an interference engagement. As such, shield assembly **40** is prevented from moving from the distal position to the proximal position, thus providing a locking mechanism for maintaining needle device **12** in the shielded position.

Shield assembly **40** may require active exertion of force thereon in order to effect movement of housing **42** from the proximal position to the distal position in the direction of arrow **80.** For example, a force may be exerted by the user on lip **56** of housing **42** in the direction of arrow **80.** Such force causes housing **42** to move with respect to hub **30** in a direction toward distal end **24** of needle cannula **20,** i.e., between a proximal position and a distal position. In particularly desirable embodiments, needle device **12** is passively shieldable, in that housing **42** automatically moves in a direction of arrow **80** to achieve secure shielding of puncture tip **28** upon release of housing **42** from the proximal position. To achieve such passive shielding, shield assembly **40** may include means for storing energy for movement of shield assembly **40** in a direction toward distal end **24** of needle cannula **20** upon release of housing **42** from the proximal position. For example, shield assembly **40** may include a torsion spring capable of exerting a biasing force to propel housing **42** from the proximal position to the distal position. Housing **42** may be releasably maintained in the proximal position through a mechanism such as a latch, which can be released to permit movement of housing **42** from the proximal position to the distal position. Desirably, the means for storing energy for movement of shield assembly **40** automatically engages to move shield assembly **40** once a force is exerted upon housing **42** in a direction of arrow **80,** such as through a user's thumb.

Blood collection set **10** can be packaged substantially in the condition shown in FIG. 1. Coil **60** provides blood collection set **10** with a profile which reduces packaging as compared with a conventional blood collection set, thereby reducing costs associated with packaging and handling of blood collection sets.

Prior to use, blood collection set **10** is removed from its package. Blood collection set **10** is provided with needle device **12** assembled and including flexible tube **14** extending from needle device **12** and connected to fixture **16,** with coil **60** in a tightly wound profile. After removing blood collection set **10** from its package, it can be assembled with other appropriate medical equipment for use. For example, fixture **16** may be connected to an appropriate receptacle for providing fluid communication with lumen **26** through needle cannula **20,** such as a non-patient needle assembly and a needle holder for use with blood collection tubes, as are known in the art.

To prepare for use of blood collection set **10,** the user grasps blood collection set **10,** with coil **60** held in the user's palm, and with needle device **12** maintained between the user's thumb and forefinger. Packaging cover **18** is then grasped and urged distally to disengage from needle cannula **20,** thereby exposing puncture tip **28** of needle cannula **20.**

The medical practitioner can then urge puncture tip **28** at distal end **24** of needle cannula **20** into a targeted blood vessel of a patient, while needle device **12** is maintained between thumb and forefinger to assist in controlled entry by the medical practitioner. During such positioning, wings **36** may be bent inwardly toward each other between the user's thumb and fingers.

After the targeted blood vessel has been accessed, the medical practitioner can release the grip. Wings **36** may then be secured to the user's skin, such as through tape, to prevent movement of needle device **12** during the procedure. The coiled nature of coil **60** of flexible tube **14** assists in preventing needle cannula **20** from accidentally pulling out of the patient's vessel. For example, in conventional blood collection systems, there is little slack in the tube extending from the needle device. As such, any movement of the patient, or extension of the tube by the user, may result in the needle cannula accidentally pulling out of the patient. Coil **60** provides flexibility to tube **14,** thereby providing more freedom of movement between the patient and the user. In addition, coil **60** maintains fixture **16** in a position adjacent the puncture site. As such, the receptacle attached to fixture **16** can be maintained adjacent the puncture site, providing for better operation by the user.

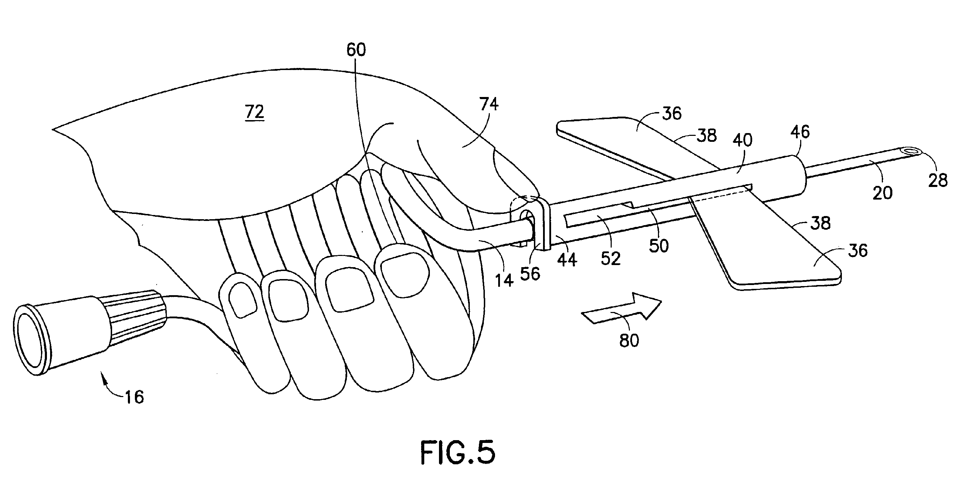

Upon completion of the procedure, such as when all desired samples have been drawn, needle cannula **20** is withdrawn from the patient. The user grasps blood collection set **10** about coil **60** as shown in FIG. 5, maintaining coil **60** within the user's hand **72,** with the user's thumb **74** pressed against lip **56** of housing **42.** After removal of needle cannula **20** from the patient, the user's thumb **74** is moved in a direction of arrow **80,** thereby forcing housing **42** in a direction of arrow **80.** The additional surface area provided to tube **14** through coil **60** provides blood collection set **10** with a profile which is particularly adaptable for fitting within the palm of a user's hand, thereby providing for simple and effective one-handed activation of the shield assembly.

During movement of housing **42** along needle cannula **20** toward the distal end **24,** wings **36** slide through grooves **50,** and into slots **52.** Interference engagement between forward edge **38** of wings **36** and shoulder **54** of slots **52** prevents housing **42** from moving in the opposing direction. Shield assembly **40** has an overall dimension that will prevent movement of housing **42** distally beyond needle cannula **20.** Hence, puncture tip **28** of needle cannula **20** is safely shielded. Blood collection set **10** may then be appropriately discarded. Coil **60** of flexible tube **14** provides blood collection assembly set **10** with an overall dimension that provides for ease of disposal as compared with conventional blood collection sets including extended flexible tubes.

While the needle assembly of the present invention has been described in terms of one embodiment for use in connection with a blood collection system, it is further contemplated that the needle assembly could be used with other medical procedures, such as in conjunction with a conventional intravenous infusion set, which are well-known in the art for use with needle assemblies.

While the present invention is satisfied by embodiments in many different forms, there is shown in the drawings and described herein in detail, the preferred embodiments of the invention, with the understanding that the present disclosure is to be considered as exemplary of the principles of the invention and is not intended to limit the invention to the embodiments illustrated. Various other embodiments will be apparent to and readily made by those skilled in the art without departing from the scope and spirit of the invention. The scope of the invention will be measured by the appended claims and their equivalents.

## Claims

1. A blood collection set comprising:
a fixture for connecting said blood collection set to a receptacle;
an intravenous needle assembly including a needle cannula having a puncture tip; and
a flexible tube extending between said fixture and said needle assembly, said flexible tube having a first end connected to said fixture and an opposed second end connected to said intravenous needle assembly, said flexible tube including a plurality of helical turns establishing a coiled structure.

2. A blood collection set according to claim 1, wherein said coiled structure includes a circular cross-section.

3. A blood collection set according to claim 1, wherein said coiled structure includes an elliptical cross-section.

4. A blood collection set according to claim 1, wherein said needle assembly includes a shield assembly adapted for movement from a first position to a second position for protectively shielding said puncture tip of said needle cannula.

5. A blood collection set according to claim 4, wherein said shield assembly comprises a housing extending about said needle cannula, said housing adapted for movement from said first position to said second position for protectively shielding said puncture tip of said needle cannula.

6. A blood collection set according to claim 5, wherein said housing includes a lip portion adapted for engagement with a user's thumb for effecting movement from said first position to said second position.

7. A blood collection set according to claim 1, wherein said needle assembly includes a hub, with said needle cannula extending from one end of said hub and said flexible tube attached to an opposing end of said hub.

8. A blood collection set according to claim 7, wherein said hub includes a pair of wings extending laterally from opposing sides of said hub.

9. A blood collection set according to claim 1, further comprising a packaging cover frictionally engaged on said needle cannula and securely surrounding said needle cannula.

10. A shieldable blood collection set according to claim 5, wherein said coiled structure is adjacent said shield assembly such that said shield assembly can be moved to said second position by a user's hand while maintaining said coiled structure in the user's hand.

11. A shieldable blood collection set according to claim 7, wherein said hub includes a pair of wings extending laterally from opposing sides of said hub.
